# EUROPÄISCHE PATENTANMELDUNG

(11) **EP 1 728 468 A1**
(43) Veröffentlichungstag der Anmeldung: **06.12.2006**
(21) Anmeldenummer: 05012091.4
(22) Anmeldetag: 04.06.2005
(51) Int. Cl.: A61B 5/00, G06F 19/00, G01N 33/48

(54) **Bewertung von Werten der Blutglucosekonzentration zur Einstellung der Insulindosierung**

(71) Anmelder: Roche Diagnostics GmbH, 68305 Mannheim (DE); F. HOFFMANN-LA ROCHE AG, 4070 Basel (CH)
(72) Erfinder: Staib, Arnulf, Dr., 64646 Heppenheim (DE); Klötzer, Hans-Martin, Dr., 68163 Mannheim (DE)
(74) Vertreter: Jany, Peter

(57) **Zusammenfassung**

Beschrieben wird ein Verfahren zur Bewertung einer Serie von Werten der Blutglucosekonzentration eines Diabetikers für eine Einstellung der Dosierung von Insulingaben, bei dem Werte der Blutglucosekonzentration g(t1) bis g(tn) für Zeitpunkte t1 bis tn, die über einen Zeitraum von mindestens vier Stunden, vorzugsweise mindestens sechs Stunden verteilt sind, erfaßt werden; auf der Grundlage der Konzentrationswerte, ein Störungsparameter ermittelt wird, der Art und/oder Schwere einer trotz Insulingaben vorhandene Störung des untersuchten Glucosestoffwechsels charakterisiert; der Störungsparameter anhand von vorgegebenen Parameterbereichen einer von mindestens zwei vorgegebenen Klassen zugeordnet wird, wobei den Klassen jeweils Empfehlungen zur Einstellung der Dosierung der Insulingaben zugeordnet sind, so daß diese Empfehlungen dem Diabetiker bereit gestellt werden können. Erfindungsgemäß ist vorgesehen, daß zum Ermitteln des Störungsparameters zunächst aus den Konzentrationswerten g(t₁) bis g(tₙ) Störungswerte w(g(t1)) bis w(g(tn)) als Funktionswerte einer Wertungsfunktion w berechnet werden und in einem weiteren Schritt aus den Störungswerten durch eine statistische Auswertung der Störungsparameter ermittelt wird. Ferner wird eine zur Durchführung dieses Verfahrens geeignete Vorrichtung beschrieben.

## Beschreibung

Die Erfindung betrifft ein Verfahren zur Bewertung einer Serie von Werten der Blutglucosekonzentration eines Diabetikers für eine Einstellung der Dosierung von Insulingaben sowie eine zur Durchführung dieses Verfahrens geeignete Vorrichtung.

Schwerwiegende langfristige Folgen des Diabetes mellitus (beispielsweise Erblinden aufgrund einer Retinopathie) können nur vermieden werden, wenn der Blutzuckerspiegel durch exakt dosierte Insulingaben ständig innerhalb enger Grenzen gehalten wird, die denen einer gesunden Person entsprechen. Insulinpflichtige Diabetiker müssen deshalb mehrmals täglich ihre Blutglucosekonzentration messen und sich Insulingaben in der jeweils benötigten Menge verabreichen.

Die optimale Dosierung von Insulingaben hinsichtlich Menge und Häufigkeit läßt sich nicht ohne weiteres aus Meßwerten der Blutglucosekonzentration ableiten. In der Praxis beruhen die gewählten Insulindosierungen zu einem erheblichen Teil auf Erfahrungswerten des behandelnden Arztes oder auch des Patienten selbst. Typischerweise wird für einen Diabetiker von einem Arzt ein Dosierungsplan erstellt, der einerseits Menge und Häufigkeit von Insulingaben zur Deckung eines Insulingrundbedarfs vorgibt und ferner Instruktionen enthält, wie als Reaktion auf erhöhte Meßwerte der Blutglucosekonzentration und Mahlzeiten zusätzliche Insulingaben dosiert werden sollen. Insulingaben zur Deckung des Insulingrundbedarfs werden in diesem Zusammenhang als Basalrate und zusätzliche Insulingaben im Zusammenhang mit Mahlzeiten als Bolus bezeichnet.

Die allgemeine Dosierungsanweisung, nach der ein Diabetiker unter Berücksichtigung von Meßwerten der Blutglucosekonzentration die Dosierung der zu verabreichenden Insulingaben ermittelt, bezeichnet man als Einstellung. Es besteht ein erheblicher Bedarf eine vorgenommene Einstellung zu bewerten, um diese gegebenenfalls korrigieren oder an eine Änderung der Lebensgewohnheiten oder des allgemeinen Gesundheitszustandes eines Patienten anpassen zu können.

Eine suboptimale Einstellung der Dosierung von Insulingaben läßt sich selbst anhand einer Serie von Meßwerten der Blutglucosekonzentration nicht ohne weiteres erkennen, da auch bei gesunden Menschen die Blutglucosekonzentration im Tagesverlauf starken Schwankungen unterliegt. In dem Artikel von N. Weintrob et al. "Glycemic patterns detected by continuous subcutaneous glucose sensing in children and adolescents with Type 1 Diabetes Mellitus treated by multiple injections vs continuous subcutaneous insulin infusion", Arch. Pediatr. Adolesc. 158, 677 (2004) wird der Ansatz verfolgt, kontinuierlich gemessene Blutglucosekonzentrationen hinsichtlich der Einstellung der Dosierung von Insulingaben anhand von Zeitintegralen der Blutglucosekonzentration zu bewerten.

Bei dieser Vorgehensweise wird für einen Zeitraum Δt von mehreren Tagen eine Fläche bestimmt, die oberhalb einer durch einen Schwellenwert von 180 mg/dl vorgegebenen Basislinie und unter der Kurve des zeitlichen Blutglucoseverlaufs liegt. Diese Fläche wird durch den Zeitraum Δt dividiert, um einen Parameter zu erhalten, der eine hyperglykämische Störung des Glukosestoffwechsels kennzeichnet. In entsprechender Weise wird ein Parameter bestimmt, der hypoklykämische Störungen des Glukosestoffwechsels kennzeichnet, indem eine Fläche zwischen einer durch einen unteren Schwellenwert von 70 mg/dl vorgegebenen Basislinie und der Kurve des zeitlichen Blutglukoseverlaufs für Zeiten bestimmt wird, zu denen die Blutglucosekonzentration diesen Schwellenwert unterschreitet.

Das bekannte Verfahren wird als Area-Under-Curve (AUC) Berechnung bezeichnet. Ein ähnliches Verfahren ist zur Diagnose von Diabetes auch aus der WO 2004/043230 A2 bekannt. Darin wird empfohlen ergänzend weitere Merkmale eines gemessenen Verlaufs der Blutglucosekonzentration, beispielsweise die Steigung, auszuwerten.

Mit derartigen Verfahren läßt sich zwar generell eine Aussage ermitteln ob ein insulinpflichtiger Diabetiker gut eingestellt ist. Wesentlich für eine gezielte Therapieempfehlung oder eine Optimierung der Einstellung ist aber die Erkennung von Perioden glykämischer Instabilitäten in dem Glucoseprofil und deren kausale Zuordnung zu einer erfolgten Insulindosierung oder Nahrungsaufnahme. Insbesondere für Personen mit einer schwankenden Insulinsensivität (sogenannte brittle Diabetes) ist es mit bekannten Verfahren praktisch nicht möglich eine optimale Einstellung der Insulindosierung zu erreichen.

Aufgabe der Erfindung ist es deshalb, einen Weg aufzuzeigen, wie eine Serie von Werten der Blutglucosekonzentration eines Diabetikers zur Einstellung der Dosierung von Insulingaben besser bewertet werden kann, so daß mit geringerem Aufwand eine optimale Insulindosierung für Diabetiker erreicht werden kann. Insbesondere soll auch bei Patienten mit schwankender Insulinsensivität durch eine verbesserte Bewertung eine Serie von Meßwerten der Blutglucosekonzentration die Einstellung der Dosierung von Insulingaben verbessert werden können.

Diese Aufgabe wird gelöst durch ein Verfahren zur Bewertung einer Serie von Werten der Blutglucosekonzentration eines Diabetikers für die Einstellung der Dosierung von Insulingaben, bei dem Werte der Blutglucosekonzentration g(t1) bis g(tn) für Zeitpunkte t1 bis tn, die über einen Zeitraum von mindestens vier Stunden, vorzugsweise mindestens sechs Stunden verteilt sind, erfaßt werden, auf der Grundlage der Konzentrationswerte der Blutglucosekonzentration, ein Störungsparameter ermittelt wird, der Art und/oder Schwere einer trotz Insulingaben vorhandene Störung des untersuchten Glucosestoffwechsels charakterisiert, der Störungsparameter anhand von vorgegebenen Parameterbereichen einer von mindestens zwei vorgegebenen Klassen zugeordnet wird, wobei den Klassen jeweils Empfehlungen zur Einstellung der Dosierung der Insulingaben zugeordnet sind, so daß diese Empfehlungen dem Diabetiker bereit gestellt werden können, dadurch gekennzeichnet, daß zum Ermitteln des Störungsparameters zunächst aus den Konzentrationswerten g(t₁) bis g(tₙ) Störungswerte w(g(t1)) bis w(g(tn)) als Funktionswerte einer Wertungsfunktion w berechnet werden und in einem weiteren Schritt aus den Störungswerten durch eine statistische Auswertung der Störungsparameter ermittelt wird.

Die Aufgabe wird ferner gelöst durch eine Vorrichtung zur Bewertung einer Serie von Werten der Blutglucosekonzentration eines Diabetikers für eine Einstellung der Dosierung von Insulingaben, umfassend eine Meßeinheit zum Messen von Meßwerten der Blutglucosekonzentration g(t1) bis g(tn) für Zeitpunkte t1 bis tn, die über einen Zeitraum von mindestens vier Stunden, vorzugsweise von mindestens sechs Stunden verteilt sind, einen Speicher zum Speichern der Konzentrationswerte, und eine Auswerteeinheit zum Auswerten der Konzentrationswerte, dadurch gekennzeichnet, daß die Auswerteeinheit so eingerichtet ist, daß sie im Betrieb durch Anwendung des erwähnten Verfahrens einen Störungsparameter ermittelt und einer von mindestens zwei vorgegebenen Klassen zuordnet, wobei den Klassen jeweils Empfehlungen hinsichtlich der optimalen Dosierung der Insulingaben zugeordnet sind, so daß diese Empfehlungen zur Einstellung der Dosierung der Insulingaben bereit gestellt werden können.

Durch die Verwendung einer Wertungsfunktion können Werte der Blutglucosekonzentration, die besonders stark von dem vorgegebenen Sollbereich, beispielsweise 70 mg/dl bis 180 mg/dl, abweichen bei der Berechnung des Störungsparameters mit einem größerem Gewicht berücksichtigt werden als Konzentrationswerte, die nur geringfügig von dem Sollbereich abweichen. Erfindungsgemäß kann deshalb auf der Grundlage von Funktionswerten der Wertungsfunktion ein Störungsparameter ermittelt werden, der eine Störung des gesuchten Glucosestoffwechsels zutreffender charakterisiert und folglich eine Verbesserung der Einstellung des Dosierung der Insulingaben ermöglicht.

In diesem Zusammenhang ist unter der Störung des untersuchten Glukosestoffwechsels selbstverständlich nicht die krankheitsbedingte Störung zu verstehen, die Insulingaben erforderlich macht, sondern die trotz der Insulingaben noch vorhandene, nicht kompensierte Störung. Der erfindungsgemäß ermittelte Störunsparameter kennzeichnet also eventuell vorhandene Unterschiede zwischen dem zeitlichen Verlauf der Blutglucosekonzentrationswerte eines durch Insulingaben behandelten Diabetikers und einer gesunden Vergleichsperson.

Wichtig ist, daß der erfindungsgemäß ermittelte Störungsparameter nicht notwendigerweiße eine eindimensionale Größe, d. h. eine natürliche oder reelle Zahl, sondern vorzugsweise ein Satz von Werten ist, die jeweils verschiedene Aspekte der Störung charakterisieren. Beispielsweise kann der Störungsparameter ein Wertepaar sein, bei dem ein erster Wert die Schwere von hyper- oder hypoglykämischen Abweichungen von dem Sollbereich und ein zweiter Wert, deren zeitliche Dauer charakterisiert. Dieses Wertepaar könnte beispielsweise um einen dritten Wert ergänzt werden, der die Häufigkeit derartiger Störungen angibt.

Besonders vorteilhaft läßt sich die Erfindung zur Bewertung von Konzentrationswerten nutzen, die über längere Zeiträume, beispielsweise mehrere Tage, in relativ kurzen Zeitabständen von wenigen Minuten gemessen wurden. Meßtechniken für entsprechende Messungen sind in der Literatur unter dem Stichwort continuous monitoring (CM) beschrieben. Steht eine ausreichend lange Serie von Konzentrationswerten, die in hinreichend kleinen Abständen gemessen wurden, zur Verfügung, so läßt sich das erfindungsgemäße Verfahren zunächst für einzelne Zeitintervalle, die in dem untersuchten Zeitraum enthalten sind, anwenden und aus den für die einzelnen Zeitintervalle ermittelten Resultaten in einem weiteren Schritt, vorzugsweise durch statistische Methoden, der Störungsparameter ermitteln.

Bei dieser Vorgehensweise wird aus Störungswerten des betreffenden Intervalls zunächst eine Störungskennzahl berechnet und in einem weiteren Schritt aus den Störungskennzahlen der einzelnen Intervalle der Störungsparameter ermittelt. Für Fälle, in denen nur eine relativ geringe Anzahl von Konzentrationswerten zur Verfügung steht und deshalb nur ein einziges Zeitintervall ausgewertet wird, kann die Störungskennzahl mit dem Störungsparameter übereinstimmen.

Im einfachsten Fall handelt es sich bei den erfindungsgemäß ausgewerteten Konzentrationswerten jeweils um Meßwerte. Diese können in geeigneter Weise, beispielsweise durch Anwendung statistischer Verfahren oder geeigneter Filteralgorithmen, aufbereitet werden, um Konzentrationswerte zu bestimmen.

Für die konkrete Form der erfindungsgemäß zu verwendeten Wertungsfunktion gibt es eine Vielzahl von Möglichkeien. Als Wertungsfunktionen können beispielsweise im Bereich von Optimierungsproblemen gebräuchliche Penalty-Funktionen verwendet werden.

Bevorzugt hat die Wertungsfunktion in einem Sollbereich der Werte der Blutglucosekonzentration eine geringere Steigung als in einem an den Sollbereich angrenzenden Bereich. Bevorzugt liegt dieser Sollbereich zwischen 50 mg/dl und 250 mg/dl, vorzugsweise zwischen 70 mg/dl und 180 mg/dl. Bei einer entsprechend gewählten Wertungsfunktion, insbesondere einer nichtlinearen Wertungsfunktion, kann der Sollbereich auch wesentlich kleiner, im Extremfall sogar als Sollwert von beispielsweise 120 mg/dl, gewählt werden.

Weitere Einzelheiten und Vorteile der Erfindung werden anhand eines Ausführungsbeispiels unter Bezugnahme auf die beigefügten Zeichnungen erläutert. Die darin offenbarten Merkmale können einzeln oder in Kombination verwendet werden, um bevorzugte Ausgestaltungen der Erfindung zu schaffen. Es zeigen:
- Fig. 1: ein Beispiel einer Wertungsfunktion;
- Fig. 2: weitere Beispiele einer Wertungsfunktion;
- Fig. 3: ein Beispiel einer Serie von Werten der Blutglucosekonzentration eines Diabetikers;
- Fig. 4: Mittelwerte und Standardabweichungen der Funktionswerte der Wertungsfunktion für überlappende Zeitintervalle von jeweils 12 Stunden für die in Fig. 3 dargestellte Serie;
- Fig. 5: Standardabweichungen und Mittelwerte von Funktionswerten der Wertungsfunktion für verschiedene Probanden; und
- Fig. 6: eine schematische Darstellung einer Vorrichtung zur Durchführung eines erfindungsgemäßen Verfahrens.

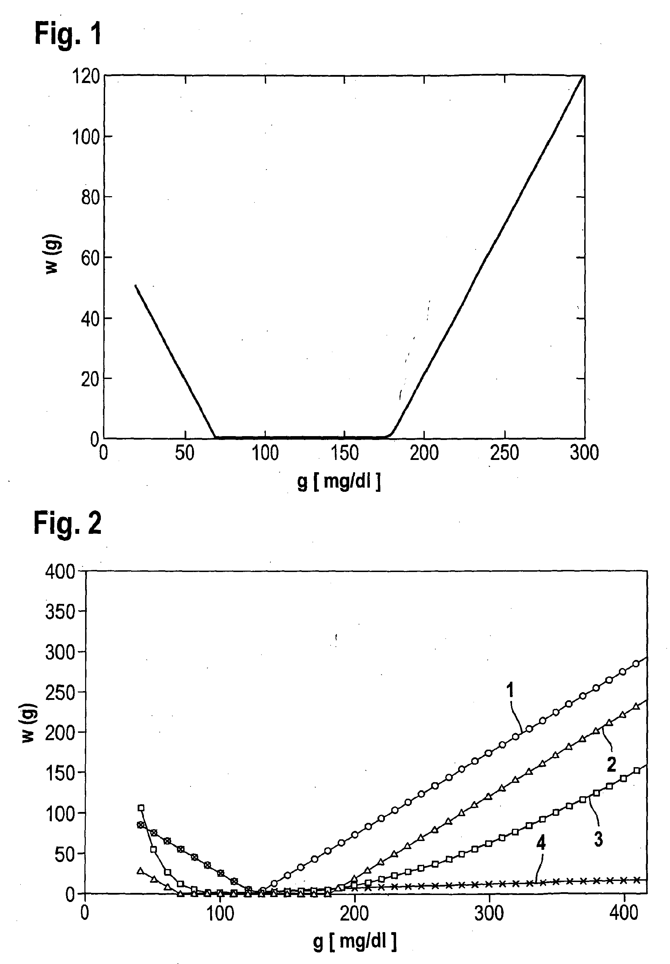

In Fig. 1 ist ein Beispiel einer Wertungsfunktion w dargestellt, mit der aus Werten der Blutglucosekonzentration g Störungswerte w(g) berechnet werden. Eine wesentliche Eigenschaft der Wertungsfunktion besteht darin, daß sie in einem Sollbereich der Blutglucosekonzentration eine geringere Steigung als außerhalb des Sollbereichs hat. Bei dem dargestellten Beispiel wurde als Sollbereich ein Normoglykämischer Bereich von 70 mg/dl bis 180 mg/dl gewählt.

Die dargestellte Wertungsfunktion steigt außerhalb des Sollbereichs linear an und hat in dem Sollbereich einen konstanten Verlauf. Es ist jedoch nicht erforderlich, daß als Wertungsfunktion eine solche abschnittsweise lineare Funktion gewählt wird. Möglich ist es auch, daß die Wertungsfunktion außerhalb des Sollbereichs nicht linear ansteigt. Auch ist nicht unbedingt erforderlich, daß die Wertungsfunktion in dem Sollbereich konstant ist. Beispielsweise kann als Wertungsfunktion auch eine parabelförmige Funktion gewählt werden, deren Scheitelpunkt sich bei etwa 120 mg/dl befindet.

Weitere Beispiele geeigneter Wertungsfunktionen sind in Fig. 2 dargestellt. Die Wertungsfunktion 1 ist ein Beispiel für eine Wertungsfunktion, bei welcher der Sollbereich stark verkleinert wurde, so daß er in dem dargestellten Extremfall zu einem Punkt zusammengeschrumpft ist. Die Funktionen 2 und 3 sind Beispiele für asymmetrische Wertungsfunktionen, die hypoglykämische Abweichungen von dem Sollbereich stärker gewichten als hyperglykämische Abweichungen. Die Wertungsfunktionen 2 und 3 sind zugleich Beispiele für nichtlineare Wertungsfunktionen. Bei der Wertungsfunktion 3 handelt es sich um den Schlichtkrull Index. Die Wertungsfunktion 4 stimmt in dem hypoglykämischen Bereich mit der Wertungsfunktion 1 überein. In dem hyperglykämischen Bereich steigt die Wertungsfunktion 4 zunächst in einem an den Sollbereich angrenzenden Abschnitt an, geht im weiteren jedoch in einen konstanten Verlauf über. Die Funktion 4 ist deshalb auch ein Beispiel dafür, daß eine Wertungsfunktion nicht überall außerhalb des Sollbereichs eine größere Steigung als innerhalb des Sollbereichs haben muß. Es genügt vielmehr, daß die Steigung in einem an den Sollbereich angrenzenden Bereich größer als in dem Sollbereich selbst ist.

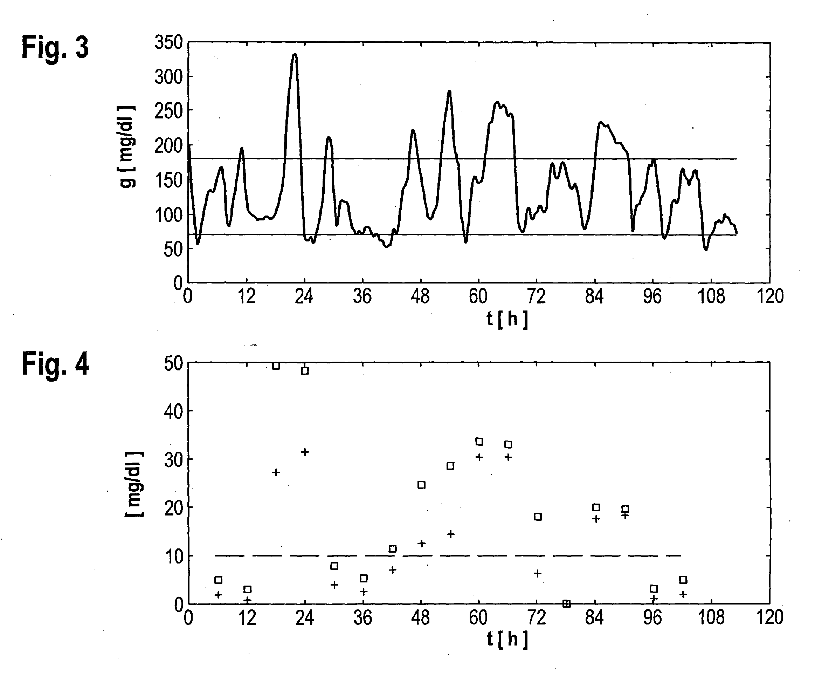

Fig. 3 zeigt ein Beispiel einer Serie von Werten der Blutglucosekonzentration eines Diabetikers über einen Zeitraum von etwa 110 Stunden. Die dargestellten Konzentrationswerte wurden in Abständen von etwa 5 Minuten ermittelt. Der Sollbereich der in Fig. 1 dargestellten Wertungsfunktion ist in Fig. 2 durch horizontal verlaufende Linien bei Blutglucosekonzentrationen von 70 mg/dl beziehungsweise 180 mg/dl angedeutet. Fig. 3 zeigt, daß die Blutglucosekonzentration im Tagesverlauf erheblichen Schwankungen unterliegt und bei einem Diabetiker sowohl hyper- als auch hypoglykämische Perioden auftreten, die durch eine optimale Einstellung der Dosierung von Insulingaben verhindert werden sollen.

Zur Auswertung wurden aus den Konzentrationswerten der in Fig. 3 dargestellten Serie zunächst Störungswerte als Funktionswerte der anhand von Fig. 1 beschriebene Wertungsfunktion berechnet. Diese Störungswerte wurden jeweils für 12 stündige Zeitintervalle ausgewertet, indem ein Mittelwert der Störungswerte des jeweiligen Zeitintervalls und die dazugehörende Standardabweichung berechnet wurden. Bevorzugt wird als Mittelwert das arithmetische Mittel gebildet.

In Fig. 4 sind die für zwölfstündige Zeitintervalle berechneten Mittelwerte der Störungswerte w(g) jeweils durch Kreuze (+) und die dazugehörenden Standardabweichungen durch kleine Kästchen (□) dargestellt. Bevorzugt werden die Zeitintervalle überlappend gewählt. Bei dem dargestellten Ausführungsbeispiel überlappen aufeinanderfolgende Zeitintervalle jeweils um 6 Stunden. Die Zeitintervalle können im Einzelfall an den Tagesablauf eines Patienten angepaßt werden, so daß sie beispielsweise mit dessen nächtlichen Schlafperioden oder Wachzeiten übereinstimmen. Günstig ist auch eine Wahl der Zeitintervalle gemäß prä- und postprandialen Phasen, so daß therapeutisch relevante Zusammenhänge leichter aufgezeigt werden können.

Die in Fig. 4 dargestellten Mittelwerte und die dazugehörenden Standardabweichungen stellen als Wertepaar eine Störungskennzahl für das jeweilige Zeitintervall dar, die Art und/oder Schwere einer Störung des untersuchten Glucosestoffwechsels charakterisiert. Dabei ist es selbstverständlich nicht zwingend, daß als Komponenten der Störungskennzahlen die Mittelwerte bzw. die Standardabweichungen selbst verwendet werden. Ebensogut läßt sich ein beliebiges Maß für die Mittelwerte bzw. die Standardabweichungen als Komponente der Störungskennzahlen verwenden. Im einfachsten Fall ist das Maß ein Vielfaches des Mittelwertes bzw. der Standardabweichung oder wird durch Addition eines konstanten Terms aus dem Mittelwert bzw. der Standardabweichung berechnet.

Aus den Störungskennzahlen der einzelnen Zeitintervalle wird durch eine statistische Auswertung ein Störungsparameter ermittelt, der einer von mindestens zwei vorgegebenen Klassen zugeordnet wird. Diesen Klassen sind jeweils Empfehlungen zur Einstellung der Dosierung der Insulingaben zugeordnet, so daß auf diese Weise die Einstellung der Dosierung der Insulingaben verbessert werden kann.

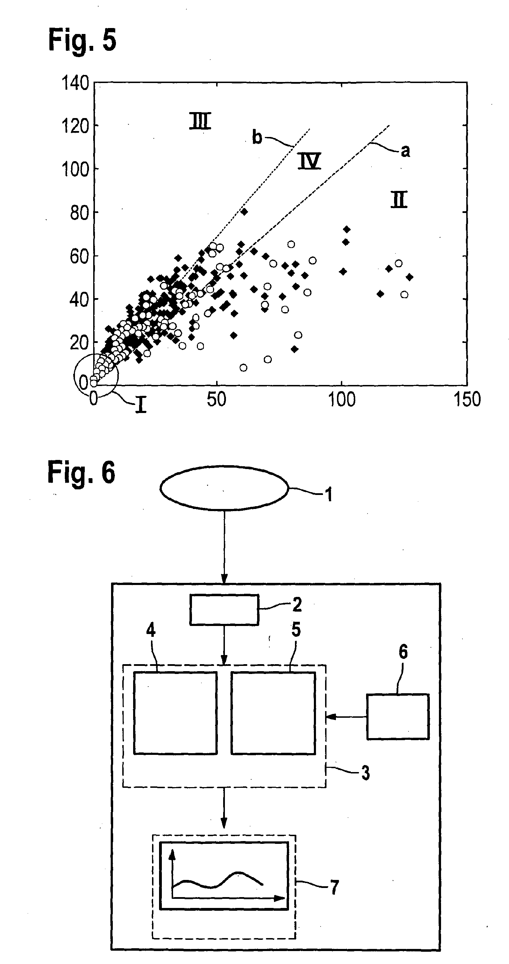

In Fig. 5 sind Standardabweichungen und Mittelwerte von Funktionswerten der Wertungsfunktion eingetragen, die auf die beschriebene Weise ermittelt wurden. Die Ordinate gibt die Standardabweichungen und die Abszisse die Mittelwerte der Funktionswerte der Wertungsfunktion für Zeitintervalle von jeweils 12 Stunden an. In Fig. 5 sind Ergebnisse der Auswertung von Serien von Blutglucosekonzentrationswerten dargestellt, die an 45 Probanden gemessen wurden. Jede Serie wurde dabei über einen Zeitraum von etwa 5 Tagen gemessen. Ergebnisse für Typ 1 Diabetiker sind dabei durch Rauten, Ergebnisse für insulinpflichtige Typ 2 Diabetiker durch Kreise dargestellt.

Fig. 5 zeigt, daß zwischen Ergebnissen für Typ 1 Diabetiker und Ergebnissen für Typ 2 Diabetiker kein signifikanter Unterschied besteht. Dies ist nicht überraschend, da bei der Einstellung der Dosierung von Insulingaben in beiden Fällen im wesentlichen die selben Schwierigkeiten auftreten. Wie anhand von Fig. 4 erläutert wurde, stellen ein Mittelwert und die dazugehörende Standardabweichung der Funktionswerte der Wertungsfunktion als Wertepaar eine Störungskennzahl für das jeweilige Zeitintervall einer untersuchten Serie von Konzentrationswerten dar. Diese Störungskennzahlen lassen sich in vier verschiedene Klassen einteilen.

Zu einer ersten Klasse I gehören Störungskennzahlen, die in Fig. 5 in einem Kreis um den Ursprung des Koordinatensystems eingezeichnet sind. Störungskennzahlen der Klasse I sind so gering, daß von einer optimalen Einstellung der Dosierung der Insulingaben ausgegangen werden kann. In diesen Fällen ist keine Änderung der Einstellung erforderlich.

Einer zweiten Klasse II werden Störungskennzahlen zugeordnet, die in Fig. 5 unterhalb einer gestrichelt dargestellten Ursprungsgraden a dargestellt sind. Zu der Klasse II gehören Fälle, in denen die Mittelwerte der Störungswerte größer sind, als die dazugehörenden Standardabweichungen. Störungskennzahlen der Klasse II deuten deshalb auf Fälle hin, in denen für längere Zeiträume eine deutliche Abweichung der Blutglucosekonzentrationswerte von dem Sollbereich aufgetreten ist. Störungskennzahlen der Klasse II lassen folglich auf eine schlecht eingestellte Basalrate der Insulingaben schließen. Handelt es sich bei der beobachteten Abweichung der Blutglucosekonzentration von dem Sollbereich um hypoglykämische Abweichungen, so ist die Basalrate zu verringern, handelt es sich um hyperglykämische Abweichungen, so ist die Basalrate zu erhöhen.

Einer dritten Klasse III werden Störungskennzahlen zugeordnet, die in Fig. 5 oberhalb einer punktiert eingezeichneten Ursprungsgraden b dargestellt sind. Störungskennzahlen der Klasse III bedeuten, daß die Standardabweichung der Funktionswerte der Wertungsfunktion, d. h. die Störungswerte, in dem untersuchten Zeitintervall von dem Mittelwert wesentlich größer ist als der Mittelwert selbst, beispielsweise 20% größer ist. Zur Klasse III gehören also Fälle, in denen die Störungswerte starken kurzfristigen Fluktuationen unterliegen. Dies bedeutet, daß die zugrunde liegende Serie von Konzentrationswerten durch kurzfristige Abweichungen von dem Sollbereich geprägt ist. In der Regel treten derartige Abweichungen im Zusammenhang mit Mahlzeiten oder körperlicher Anstrengung auf, so daß zur Optimierung der Einstellung der Boli, also die zusätzlichen Insulingaben im Zusammenhang mit Mahlzeiten oder körperlicher Betätigung, angepaßt werden sollte.

Einer vierten Klasse IV werden Störungskennzahlen zugeordnet, die in Fig. 5 zwischen der punktierten Ursprungsgeraden b und der gestrichelten Ursprungsgeraden a dargestellt sind. In derartigen Fällen sind die Mittelwerte und die Standardabweichungen der Funktionswerte der Wertungsfunktion in dem betrachteten Zeitintervall etwa gleich groß, so daß sowohl die Basalrate als auch der Bolus der Insulingaben zu überprüfen und anzupassen ist.

Bei dem anhand von Fig. 5 erläuterten Beispiel stellt der Index der jeweiligen Klasse (d. h. die Zahl I, II, III oder IV) den Störungsparameter dar, der durch eine statistische Auswertung der Störungswerte ermittelt wurde. Jedem dieser Störungsparameter, d. h. jeder dieser Klassen, ist jeweils eine qualitative Empfehlung zugeordnet, wie die Einstellung der Dosierung von Insulingaben anzupassen ist. Werden zusätzlich zu einer Serie von Konzentrationswerten weitere für den zeitlichen Verlauf der Blutglucosekonzentration relevante Daten, insbesondere über verabreichte Insulingaben und aufgenommene Broteinheiten, erfaßt und ausgewertet, können unter Verwendung dieser Daten und der den Klassen zugeordneten Empfehlungen auch die benötigten Insulindosen und die Zeitpunkte, zu denen sie verabreicht werden sollen, berechnet werden. Dazu sind die Abweichungen der Blutglucosekonzentrationswerte von dem Sollbereich quantitativ einzubeziehen. Alternativ können die Störungskennzahlen für die jeweilige Klasse zunächst qualitativ ausgewertet werden, wobei anschließend der Grad der Abweichung erfaßt und zur Berechnung der Insulindosen herangezogen werden kann.

Bei dem im Vorhergehenden beschriebenen Verfahren wurde der Störungsparameter auf der Grundlage der Mittelwerte der Störungswerte und der Standardabweichung der Störungswerte berechnet. Ergänzend oder alternativ kann der Störungsparameter oder einige seiner Komponenten beispielsweise auch dadurch ermittelt werden, daß die Störungswerte jeweils einem von mehreren Stoffwechsel-Zuständen zugeordnet werden und ermittelt wird, wie lange die verschiedenen Zustände jeweils andauern und/oder mit welcher Häufigkeit Zustandswechsel auftreten. Eine Komponente des Störungsparameters kann dann beispielsweise ein Maß für die Zeitspanne sein, über die der betreffende Zustand andauert. Eine weitere Komponente des Störungsparameters kann ein Maß für die Häufigkeit der Zustandswechsel sein.

Störungswerte können Zuständen des Glucosestoffwechsels beispielsweise anhand von vorgegebenen Schwellenwerten zugeordnet werden. Anstelle einer Störungsfunktion, wie sie in den Figuren 1 und 2 dargestellt ist, kann bei einer derartigen Vorgehensweise auch eine Stufenfunktion verwendet werden, die beispielsweise in einem Sollbereich den Wert 0 (normoglykämischer Bereich), unterhalb des Sollbereichs den Wert -1 (hypoglykämischer Bereich) und oberhalb des Sollbereichs einen Wert von +1 hat (hyperglykämischer Bereich) hat.

Übersteigt die Anzahl der Zustandswechsel in einem betrachteten Zeitintervall einen vorgegebenen Schwellenwert, so deutet dies auf eine Instabilität in der Einstellung der Insulingaben hin und sollte deshalb in den Störungsparameter eingehen. Beispielsweise kann der Störungsparameter zusätzlich einen Wert enthalten, der die Anzahl der Zustandswechsel angibt oder aus dieser Anzahl berechnet wird.

Das beschriebene Verfahren kann von einem Analysehandgerät eingesetzt werden, mit dem Diabetiker selbständig ihren Blutzuckerspiegel überwachen. Auf diese Weise können einem Diabetiker durch das Handgerät wertvolle Hinweise gegeben werden, wie die erforderlichen Insulingaben besser an bestehende Bedürfnisse angepaßt werden können. Insbesondere kann ein Diabetiker durch Verwendung eines Handgeräts, welches das beschriebene Verfahren nutzt, selbständig kleinere Anpassungen der Einstellung vornehmen und - sofern größere Einstellungen erforderlich sind - an einen Arzt verwiesen werden.

In Fig. 6 sind die wesentlichen Komponeten eines solchen Geräts dargestellt. Eine Meßeinheit 1 mißt mit einem Sensor zu aufeinanderfolgenden Zeitpunkten tₙ Meßwerte. Dieses Meßsignal wird - im dargestellten Fall drahtlos - an einen Empfänger 2 übertragen von dem das Meßsignal an eine Auswerteeinheit 3 weitergeleitet wird, die einen Mikroprozessor 4 und einen Datenspeicher 5 enthält. Daten, beispielsweise über verabreichte Insulingaben oder aufgenommene Broteinheiten, und Befehle können auch über eine Eingabeeinheit 6 an die Auswerteeinheit übermittelt werden. Die Ausgabe von Resultaten erfolgt mittels einer Ausgabeeinheit 7, die ein Display und andere übliche Ausgabemittel einschließen kann. Selbstverständlich erfolgt die Datenverarbeitung der Auswerteeinheit 3 digital und es sind entsprechende Wandler zur Umwandlung analoger Signale in digitale Signale vorgesehen. Besonders gut sind implantierbare Sensoren geeignet, mit denen in relativ kurzen Zeitabständen, beispielsweise 5 min, Werte der Blutglucosekonzentration ermittelt werden können.

Das beschriebene Verfahren kann auch für eine Vorrichtung verwendet werden, die zusätzlich zu der Meßeinheit 2, dem Speicher 5 und der Auswerteeinheit 3 eine Insulinpumpe umfaßt, die von der Auswerteeinheit 3 unter Berücksichtigung der Empfehlung der ermittelten Klasse, welcher der Störungsparameter zugeordnet wurde, gesteuert wird.

Anstelle einer Insulinpumpe können auch andere Geräte zur Insulingabe verwendet werden, beispielsweise sogenannte Insulinpens, bei denen es sich um Injektionsgeräte von der Größe eines Kugelschreibers handelt, Bevorzugt wird die verabreichte Insulindosis drahtlos von dem zur Insulinabgabe verwendeten Gerät, beispielsweise die Insulinpumpe oder einem Insulinpen, an die Auswerteeinheit 3 übertragen. Dies kann beispielsweise nach jeder Insulingabe oder nach voreingestellten Zeitintervallen geschehen. Ergänzend können weitere Peripheriegeräte verwendet werden, beispielsweise Geräte, in denen Informationen über die Broteinheiten verschiedener Speisen gespeichert sind, so daß entsprechende Schätzwerte für eine eingenommene Mahlzeit auf Abruf an die Auswerteeinheit 3 des beschriebenen Geräts übertragen werden können. Selbstverständlich können entsprechende Informationen auch in einem Speicher der Auswerteeinheit 3 gespeichert sein.

## Patentansprüche

1. Verfahren zur Bewertung einer Serie von Werten der Blutglucosekonzentration eines Diabetikers für eine Einstellung der Dosierung von Insulingaben, bei dem:
- Werte der Blutglucosekonzentration g(t1) bis g(tn) für Zeitpunkte t1 bis tn, die über einen Zeitraum von mindestens vier Stunden, vorzugsweise mindestens sechs Stunden verteilt sind, erfaßt werden,
- auf der Grundlage der Konzentrationswerte, ein Störungsparameter ermittelt wird, der Art und/oder Schwere einer trotz Insulingaben vorhandene Störung des untersuchten Glucosestoffwechsels charakterisiert,
- der Störungsparameter anhand von vorgegebenen Parameterbereichen einer von mindestens zwei vorgegebenen Klassen zugeordnet wird, wobei den Klassen jeweils Empfehlungen zur Einstellung der Dosierung der Insulingaben zugeordnet sind, so daß diese Empfehlungen dem Diabetiker bereit gestellt werden können,
**dadurch gekennzeichnet, daß**
zum Ermitteln des Störungsparameters zunächst aus den Konzentrationswerten g(t₁) bis g(tₙ) Störungswerte w(g(t1)) bis w(g(tn)) als Funktionswerte einer Wertungsfunktion w berechnet werden und
in einem weiteren Schritt aus den Störungswerten durch eine statistische Auswertung der Störungsparameter ermittelt wird.

2. Verfahren nach Anspruch 1, **dadurch gekennzeichnet, daß** die Wertungsfunktion in einem Sollbereich der Blutglucosekonzentration eine geringere Steigung als in einem an den Sollbereich angrenzenden Bereich hat

3. Verfahren nach Anspruch 1 oder 2, **dadurch gekennzeichnet, daß** der Störungsparameter eine mehrdimensionale Größe mit mehreren Komponenten ist.

4. Verfahren nach einem der vorhergehenden Ansprüche, **dadurch gekennzeichnet, daß** die Berechnung des Störungsparameters einen Schritt umfaßt, in dem ein Mittelwert der Störungswerte berechnet wird.

5. Verfahren nach Anspruch 4, **dadurch gekennzeichnet, daß** die Berechnung des Störungsparameters einen Schritt umfaßt, in dem eine Standardabweichung der Störungswerte von dem Mittelwert berechnet wird.

6. Verfahren nach Anspruch 5, **dadurch gekennzeichnet, daß** eine Komponente des Störungsparameters ein Maß für die Standardabweichung der Störungswerte von dem Mittelwert ist.

7. Verfahren nach einem der Ansprüche 4 bis 6, **dadurch gekennzeichnet, daß** eine Komponente des Störungsparameters ein Maß für den Mittelwert der Störungswerte ist.

8. Verfahren nach einem der vorhergehenden Ansprüche, **dadurch gekennzeichnet, daß** die Berechnung des Störungsparameters einen Schritt umfaßt, in dem die Störungswerte jeweils einem von mehreren Zuständen zugeordnet werden und ermittelt wird, wie lange die verschiedenen Zustände jeweils andauern und/oder mit welcher Häufigkeit Zustandswechsel auftreten.

9. Verfahren nach Anspruch 8, **dadurch gekennzeichnet, daß** eine Komponente des Störungsparameters ein Maß für die Zeitspanne ist, über die einer der Zustände andauert.

10. Verfahren nach Anspruch 8 oder 9, **dadurch gekennzeichnet, daß** eine Komponente des Störungsparameters ein Maß für die Häufigkeit der Zustandswechsel ist.

11. Verfahren nach einem der vorhergehenden Ansprüche, **dadurch gekennzeichnet, daß** für mehrere Zeitintervalle, die jeweils in dem Zeitraum enthalten sind, jeweils aus den Störungswerten des betreffenden Intervalls eine Störungskennzahl berechnet wird und in einem weiteren Schritt aus den Störungskennzahlen der Störungsparameter ermittelt wird.

12. Verfahren nach Anspruch 11, **dadurch gekennzeichnet, daß** die Zeitintervalle, für welche die Störungskennzahlen jeweils berechnet werden, überlappen.

13. Verfahren nach einem der vorhergehenden Ansprüche, **dadurch gekennzeichnet, daß** die Wertungsfunktion, mit der die Störungswerte berechnet werden, eine abschnittsweise lineare Funktion ist.

14. Verfahren nach Anspruch 13, **dadurch gekennzeichnet, daß** die Wertungsfunktion in dem Sollbereich konstant ist.

15. Verfahren nach einem der vorhergehenden Ansprüche, **dadurch gekennzeichnet, daß** der Sollbereich zwischen 50 mg/dl und 250 mg/dl, vorzugsweise zwischen 70 mg/dl und 180 mg/dl, liegt.

16. Vorrichtung zur Bewertung einer Serie von Werten der Blutglucosekonzentration eines Diabetikers für eine Einstellung der Dosierung von Insulingaben, umfassend
- eine Meßeinheit (1) zum Ermitteln von Werten der Blutglucosekonzentration g(t1) bis g(tn) für Zeitpunkte t1 bis tn, die über einen Zeitraum von mindestens vier Stunden, vorzugsweise von mindestens sechs Stunden, verteilt sind,
- einen Speicher (5) zum Speichern der Konzentrationswerte, und
- eine Auswerteeinheit (3) zum Auswerten der Konzentrationswerte, **dadurch gekennzeichnet, daß**
die Auswerteeinheit (3) so eingerichtet ist, daß sie im Betrieb durch Anwendung eines Verfahrens nach einem der vorhergehenden Ansprüche einen Störungsparameter ermittelt und einer von mindestens zwei vorgegebenen Klassen zuordnet, wobei den Klassen jeweils Empfehlungen hinsichtlich der optimalen Dosierung der Insulingaben zugeordnet sind, so daß diese Empfehlungen zur Einstellung der Dosierung der Insulingaben bereit gestellt werden können.

17. Vorrichtung nach Anspruch 12 oder 13, **dadurch gekennzeichnet, daß** die Vorrichtung eine Eingabeeinheit (6) aufweist, über die für den zeitlichen Verlauf der Blutglucosekonzentration relevante Daten, insbesondere verabreichte Insulingaben und aufgenommene Broteinheiten, eingegeben oder von einem anderen Gerät empfangen werden können und die Auswerteeinheit (3) unter Verwendung dieser Daten und der den Klassen zugeordneten Empfehlungen benötigte Insulindosen berechnet.

18. Vorrichtung nach Anspruch 17, **dadurch gekennzeichnet, daß** sie eine Ausgabeeinheit (7) zum Ausgeben einer Empfehlung hinsichtlich der Dosierung der Insulingabe des Diabetikers umfaßt.

19. Vorrichtung nach Anspruch 16 oder 17, **dadurch gekennzeichnet, daß** sie eine Insulinpumpe umfaßt, die von der Auswerteeinheit (3) unter Berücksichtigung der Empfehlung der Klasse, welcher der Störungsparameter zugeordnet wurde, gesteuert wird.
